# EUROPEAN PATENT SPECIFICATION

(11) **EP 0 389 019 B1**
(45) Date of publication and mention of the grant of the patent: **10.11.1993**
(21) Application number: 90200500.8
(22) Date of filing: 02.03.1990
(51) Int. Cl.: A01H 4/00

(54) **Method for producing culture vessels filled with micro-cultures of plant parts, and device for carrying out said method**
Verfahren zur Produktion von Kulturgefässen, gefüllt mit Mikrokulturen von Pflanzenteilen, und Gerät für dieses Verfahren
Procédé de production de boîtes de culture remplies de microcultures de parties de plantes et appareil pour ce procédé

(30) Priority: 07.03.1989 NL 8900555
(43) Date of publication of application: 26.09.1990
(73) Proprietor: PLANT PRODUCTION SYSTEMS B.V., 5705 BZ Helmond (NL)
(72) Inventor: Janus, Edwin Otto Maria, NL-5674 SG Gerwen (NL)
(74) Representative: Timmermans, Anthonius C.Th., Ir.

(56) References cited:
- EP-A- 0 232 628
- WO-A-88/06618
- US-A- 3 407 120
- US-A- 4 728 135
- US-A- 4 741 196

## Description

The invention relates to a method for producing culture vessels filled with micro-cultures of plant parts in an aseptic and sterile environment, whereby a culture/growth medium for a desired culture is prepared and brought to a desired temperature, that plants previously grown from selected plant parts are cut into plants parts and the culture vessel in question filled with plant parts is sealed. Such a method is known from WO 88/006618. The known method provides however no means for filling the culture wessel with cut and selected fragile plant parts in an asptic and sterile encasement.

With the known method use is made of manpower to an extent of about 60%, the workers being provided with special clothing in order to reduce the risk of infection to a minimum. The increasing demand for the cultures of a wide variety of plants has made it economically essential to adapt to the rapidly changing and increasing demand. The necessary use of a great deal of manpower is an obstacle in this respect, however.

The invention also relates to a device for carrying out the method, whereby the device includes picking up and placing means, said picking up and placing means having at least one suction nozzle.

Such a device is known from EP-A-232628, wherein plantlets are sucked up and held by transport means, positioned over a culture medium and ejected onto the culture medium. The known device is however not suited for the purpose of picking up and placing delicate and small sized microcultures of plant parts in the medium.

The purpose of the invention is to provide a method and a device for carrying out said method, whereby the cost price of the final product is limited and is less dependent on the use of manpower, and moreover to create the possibilities to bring production to a level of massproduction, while simulaneously improving the quality of the final product.

In order to accomplish that object the method according to the invention is characterized in that water is sterilized, that the medium and the water are mixed in such a ratio that the mixture of water and medium in the culture vessel in question takes a temperature in which plant parts selected for culture will thrive, that the cut plant parts are selected, that the selected plant parts are transported unto the culture vessel in question by being carried on an air flow which compensated gravity, and by directing an air blast tranversely to the air flow, that the selected plant parts are sucked up and held after their transport, and that the selected plant parts held are positioned above and placed in the mixture of water and medium.

The advantage of the method according to the invention is that the production time of the final product has been substantially reduced, as a result of which not only the numbers of cultures produced have strongly increased, but also the quality has been improved, since the risk of infections of the delicate micro cultures produced has been reduced to a minimum.

Furthermore it is advantageous that crops that were previously economically unremunerative, because of their extra sensitivity to infective diseases or because of the very labour-intensive processing they required, have become exploitable as a result of the application of the method according to the invention, as a result of which the diversity of the ranges of cultures to be put on the market has been increased and, moreover, said wider range has become available to a larger group of buyers, on account of the lower cost of production. This last-mentioned advantage is e.g. of particular importance when we consider the world food situation with regard to the available quantity and the diversity of resistant rice plants in e.g. the developing countries. On a macro-scale we can moreover think of the fact that plants of fast-growing tree species will become available on a large scale, which may contribute towards slightly limiting the deforestation in the world.

By using air, sterilized air in practice, as a carrying and transporting medium for the selected plant exposed to mechanical forces, as a result of which the risk of injury is extremely small. Moreover, the advantage of the method according to the invention is that the delicate plant parts need not be touched by the human hand or by e.g. tweezers. Also as a result of this the risk that infections are transmitted to the plant parts will become extremely small.

A preferential embodiment of the method according to the invention is characterized in that each selected plant part is blown into said mixture of water and medium.

The advantage of the preferential embodiment of the method according to the invention is, that also at this stage of the method the selected plant part is not touched by the human hand, nor is it held by being clamped between e.g. two fingers of a gripper. Once more, at this stage of the method the risks of mechanical injury and of infection are minimal.

A simple device according to the invention for carrying out the method according to the invention is characterized in that the suction nozzle compressed air means that can switched on, that each suction nozzle includes an inner tube and an outer tube which coaxially surrounds the inner tube, and that the inner tube is coupled to the suction means and the outer tube is coupled to the compressed air means.

Test have shown that such a coaxial construction of a suction nozzle produces very favourable results.

The advantage of the device according to the invention manifests itself especially when several suction nozzles are used, whereby several selected plant parts can be placed in the mixture of water and medium at the same time. As a result of this the eventual proceeds of the method are considerably increased.

The invention will be further explained with reference to the following drawings.

Figure 1 is a diagrammatic illustration of the method and the device according to the invention.

Figure 2 is an embodiment of a coaxial suction nozzle for application in the robot of the device according to Figure 1 for picking up and placing plant parts.

Figure 3 is an embodiment of transport means for a sterile transport of selected plant parts from cutting tables to a position near the robot according to Figure 1.

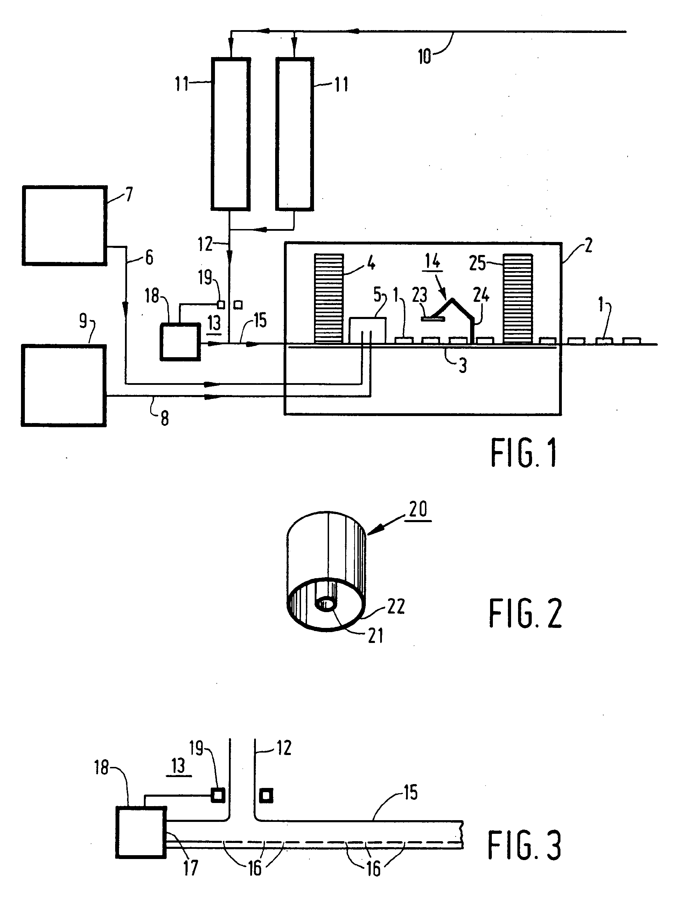

Figure 1 diagrammatically illustrates a method and device with which culture vessels 1 filled with micro-cultures of plant parts can be produced. Inside an aseptic and sterile encasement 2 there is arranged a belt, 3, which moves from the left to the right in said Figure. From a storage unit 4 the culture vessels 1 are placed on the moving belt 3. The culture vessels are sterilized and made of e.g. a plastic material, whether or not transparent, such as polyethylene, polypropylene, polycarbonate etc. Preferably they have a rectangular shape, which is preferably such that several culture vessels can be placed inside one another. After a culture vessel 1 has been placed on the moving belt it will reach a filling unit 5, diagrammatically illustrated in the Figure. The filling unit 5 is connected, via a pipe 6, to a container 7, in which a sterile culture fluid, usually water, is stored, and it is also connected, via a pipe 8, to a container 9 containing a breeding/growth medium which is suitable for the growing elected.

The fluid in the container 7 preferably has a temperature of about 2° C, and the medium in the container 9 preferably has a temperature of 55° C.

The filling unit 5 admits the fluid from the container 7 and the medium from the container 9 in a desired ratio to the culture vessel 1 in question. The ratio will particularly be determined by the desired concentration of the mixture of water and medium in the breeding vessel 1, as well as by the desired final temperature of the mixture in the relevant culture vessel 1. The mixture preferably has a temperature value which lies within the range from about 25° C to about 30° C. The temperature of the mixture in the culture vessel 1 is such that the plant parts, in case they are dipped in the mixture, do not sustain any damage by a possible sudden change of temperature. The plants previously raised from selected plant parts are supplied via the line in order to be processed and selected on cutting tables 11. Then the selected plant parts are taken, via air line 12, to a special device 13 for the transport of selected plant parts to a robot 14, which is placed inside the encasement 2.

First the device 13 will be further explained with reference to Figure 3. The device 13 substantially includes a tube 15 having a series of openings 16, which are located in the longitudinal direction, spaced by regular distances from one another. One end 17 of the tube 15 is connected to compressed air means 18. The compressed air means cause a generally continuous air flow through the openings 16 in the tube 15 and form a uniformly divided air bed therein, on which the selected plant parts land after having reached the tube 15 through the air line 12. During their passage through the air line 12 the plant parts pass a photoelectric device 19, which sends a control signal to the compressed air means 18 to effect an air blast through the tube 15 from the end 17, as a result of which the selected plant parts are moved toward the other end of the tube 15, in the direction of the robot 14, without the plant parts touching the interior of the tube 15. The selected plant parts are fully embedded in air, also during their transport, as a result of which the risks of mechanical injury and infection are reduced to a minimum.

After having passed the tube 15 in the direction of the other end thereof (not shown), the selected plant parts are sucked up by means of air and held against a suction nozzle, to be explained in more detail, of the robot 14. A possible embodiment of such a suction nozzle may comprise a simple tube, through which air flows, which sucks up the selected plant part and holds it against said suction nozzle. After the robot 14 has then been brought to a position above the culture vessel 1 in question the plant part is released, e.g. in that air is pressed through the tube. A preferential embodiment of a suction nozzle 20 is shown in Figure 2. The suction nozzle 20 has an inner tube 21 and an outer tube 22 which coaxially surrounds the inner tube 21. After a selected plant part has been detected the suction nozzle 20 is moved toward the other end of the tube 15, whereby air is sucked into the inner tube 21, as a result of which the plant part moves toward the suction nozzle 20 and is held against the end of the inner tube 21. Generally several suction nozzles, e.g. of the type shown in Figure 2, will be mounted on a plate 23 which is fixed to an arm 24 of the robot 14. With such a multiple suction nozzle the various suction nozzles are generally to be provided with air pressure separately. When all suction nozzles 20 are provided with a selected plant part the arm 24 is moved to a position above the relevant culture vessel containing the mixture, and subsequently air is blown between the inner tube 21 and the outer tube 22, possibly after the passage of air through the inner tube 21 has been stopped. As a result of this the various selected plant parts will become detached from the suction nozzle, fall into the mixture and, as a result of the continued air pressure, be dipped therein. A storage unit 25 containing lids then takes care of it that the culture vessel 1 in question is sealed hermetically, after which the filled culture vessels are transported to a growing room. After having stayed in the growing room for some time the culture vessels are distributed, whereby part of the contents of the culture vessels is used for processing and selection on the cutting tables 11, however. It is furthermore noted that the air which is used will also be sterile, of course.

With the method and the device as explained above it is possible to save about 50% on the cost of labour, and the production capacity can be increased in a simple manner to e.g. 30 million plants per year.

## Claims

1. Method for producing culture vessels (1) filled with micro-cultures of plant parts in an aseptic and sterile enviroment, whereby a culture/growth medium for a desired culture is prepared and brought to a desired temperature, plants previously grown from selected plant parts are cut into plant parts and the culture vessel (1) in question filled with plant parts is sealed, characterized in that water is sterilized, that the medium and the water are mixed in such a ratio that the mixture of water and medium in the culture vessel (1) in question takes a temperature in which plant parts selected for culture will thrive, that the cut plant parts are selected, that the selected plant parts are transported unto the culture vessel (1) in question by being carried on an air flow which compensates for gravity, and by directing an air blast transversely to the air flow, that the selected plant parts are sucked up and held after their transport, and that the selected plant parts held are positioned above and placed in the mixture of water and medium.

2. Method according to claim 1, characterized in that by mixing the water and the medium the mixture of water and medium in the culture vessel (1) in question is given a temperature which lies within the range from about 25°C to about 30°C.

3. Method according to claim 1 or 2, characterized in that the medium is brought to a desired tempreature of 55°C and the water is brought to a desired temperature of 2°C.

4. Method according to claim 1, 2 or 3, characterized in that each selected plant part is blown into said mixture of water and medium.

5. Device for carrying out the method according to claims 1-4, whereby the device includes picking up and placing means (14), said picking up and placing means (14) having at least one suction nozzle (20), characterized in that the suction nozzle (20) is coupled to suction/compressed air means that can be switched on, that each suction nozzle (20) includes an inner tube (21) and an outer tube (22) which coaxially surrounds the inner tube (21), and that the inner tube (21) is coupled to the suction means and the outer tube (22) is coupled to the compressed air means.

## Patentansprüche

1. Verfahren zur Herstellung in einer aseptischen und sterilen Umgebung von mit Mikro-Zuchtkulturen von pflanzlichen Teilen gefüllten Zuchtgefäßen (1), wodurch ein Zucht/Wachstumsmedium für eine gewünschte Zuchtkultur bereitet wird und auf eine gewünschte Temperatur gebracht wird, vorher aus ausgewählten Pflanzenteilen gezüchtete Pflanzen zu Pflanzenteilen geschnitten werden und das betreffende Zuchtgefäß (1) mit Pflanzenteilen gefüllt luftdicht verschlossen wird, dadurch gekennzeichnet, daß Wasser sterilisiert ist, daß das Medium und das Wasser von einem derartigen Verhältnis gemischt sind, daß das Gemisch aus Wasser und Medium in dem betreffenden Zuchtgefäß (1) eine Temperatur annimmt, in der für die Zucht ausgewählte Pflanzenteile gedeihen, daß die geschnittenen Pflanzenteilen ausgewählt werde, daß die ausgewählten Pflanzenteilen bis zu dem betreffenden Zuchtgefäß (1) befördert werden, indem sie auf einem die Schwerekraft aufhebenden Luftstrom getragen werden und indem sie von einem quer zu dem Luftstrom gerichteten Luftstoß befördert werden, daß die ausgewählten Pflanzenteile nach ihrem Transport aufgesaugt und festgehalten werden und daß die festgehaltenen ausgewählten Pflanzenteile über dem Wasser/Medium-Gemisch positioniert werden und in das Wasser/Medium-Gemisch eingesetzt werden.

2. Verfahren nach Anspruch 1, dadurch gekennzeichnet, daß durch die Zusammenfügung des Wassers und des Mediums ein Wasser/Medium-Gemisch in dem betreffenden Zuchtgefäß (1) eine Temperatur erhält, die in dem Temperaturwertbereich von etwa 25°C bis etwa 30°C gelegen ist.

3. Verfahren nach Anspruch 1 oder 2, dadurch gekennzeichnet, daß das Medium auf eine gewünschte Temperatur von 55°C und das Wasser auf eine gewünschte Temperatur von 2°C gebracht wird.

4. Verfahren nach Anspruch 1, 2 oder 3, dadurch gekennzeichnet, daß jeder ausgewählte Pflanzenteil in das Wasser/Medium-Gemisch hineingeblasen wird.

5. Vorrichtung zur Durchführung des Verfahrens nach Anspruch 1 bis 4, dadurch gekennzeichnet, daß die Vorrichtung Aufnahme- und Abstellmittel (14) umfaßt, wobei die Aufnahme- und Abstellmittel (14) wenigstens einen Saugmund (20) aufweisen, dadurch gekennzeichnet, daß der Saugmund (20) mit einschaltbaren Saug-/Druckluftmitteln verbunden ist, daß jeder Saugmund (20) ein Innenrohr (21) und ein koaxial um das Innenrohr (21) befindliches Außenrohr (22) aufweist, und daß das Innenrohr (21) mit den Saugmitteln gekoppelt ist und das Außenrohr (22) mit den Druckluftmitteln gekoppelt ist.

## Revendications

1. Procédé de production, dans un entourage aseptique et stérile, de boîtes de culture (1) remplies de microcultures de parties de plantes, par lequel un moyen de culture/de croissance est préparé et porté à une température désirée, des plantes antérieurement cultivées à partir de parties de plantes sélectionnées sont coupées en des parties de plantes et la boîte (1) concernée remplie de parties de plantes est fermée hermétiquement, caractérisé en ce que l'eau est stérilisée, en ce que le moyen et l'eau sont mélangés dans un tel rapport que le mélange de l'eau et du moyen dans la boîte de culture concernée (1) prend une température à laquelle les parties de plantes sélectionnées pour la culture se plaisent, en ce que les parties de plantes coupées sont sélectionnées, en ce que les parties de plantes sélectionnées sont transportées jusqu'à la boîte de culture (1) concernée du fait qu'elles sont portées par un courant d'air compensant la pesanteur et du fait qu'elles sont transportées par un coup d'air transversal par rapport au courant d'air, en ce que les parties de plantes sélectionnées sont aspirées et retenues après leur transport, et en ce que les parties de plantes retenues sont positionnées au-dessus du mélange de l'eau et du moyen et posées dans le mélange de l'eau et du moyen.

2. Procédé selon la revendication 1, caractérisé en ce qu'en mêlant l'eau et le moyen, le mélange de l'eau et du moyen dans la boîte de culture (1) concernée est porté à une température située dans le domaine de valeurs de température de 25°C à 30°C environ.

3. Procédé selon la revendication 1 ou 2, caractérisé en ce que le moyen est porté à une température désirée de 55°C et l'eau est portée à une température désirée de 2°C.

4. Procédé selon la revendication 1, 2 ou 3, caractérisé en ce que chacune des parties de plantes sélectionnées est soufflée dans le mélange de l'eau et du moyen.

5. Appareil pour la réalisation du procédé selon les revendications 1 à 4, caractérisé en ce que l'appareil comprend des moyens d'accueil et de positionnement (14), les moyens d'accueil et de positionnement (14) comprenant au moins une bouche d'aspiration (20), caractérisé en ce que la bouche d'aspiration (20) est liée à des moyens d'aspiration/à air comprimé, en ce que chaque bouche d'aspiration comprend un tuyau intérieur (21) et un tuyau extérieur (22), disposé d'une manière coaxiale par rapport au tuyau intérieur (21), et en ce que le tuyau intérieur (21) est relié aux moyens d'aspiration et le tuyau extérieur (22) est relié aux moyens à air comprimé.
